(19) Europäisches Patentamt
European Patent Office
Office européen des brevets

(11) **EP 4 134 972 A1**

(12) **EUROPEAN PATENT APPLICATION**

(43) Date of publication:
**15.02.2023  Bulletin 2023/07**

(21) Application number: **21191175.5**

(22) Date of filing: **13.08.2021**

(51) International Patent Classification (IPC):
**G16H 30/40** (2018.01)    **G16H 40/63** (2018.01)
**G06N 3/08** (2000.01)

(52) Cooperative Patent Classification (CPC):
**G16H 30/40; G16H 40/63;** G06N 20/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **SEVENSTER, Merlijn**
  **5656 AE Eindhoven (NL)**
- **YOUNG, Stewart**
  **5656 AE Eindhoven (NL)**

(74) Representative: **Philips Intellectual Property & Standards**
**High Tech Campus 52**
**5656 AG Eindhoven (NL)**

(54)     **MACHINE LEARNING BASED QUALITY ASSESSMENT OF MEDICAL IMAGERY AND ITS USE IN FACILITATING IMAGING OPERATIONS**

(57)     System (SYS) and related method for imaging support. The system comprises an input interface (IN) for receiving an input image (11) of a patient acquired by an imaging apparatus (IA). The input image was previously assessed by an image quality assessment module (IQM) and was awarded an image quality, IQ, score. The system's imaging triaging logic (TL) retrieves from an image database (DBI) of prior assessed images, a corresponding image (10) that corresponds to the input image.

The corresponding image is a prior image of patient. The imaging triaging logic (TL) provides a decision, based on the first IQ score and a second IQ score of the corresponding image (10), if any, whether or not to acquire a new image (12) of the patient by the imaging apparatus (IA). The risk of unnecessary retakes can be reduced, thus saving time, dose, and reducing wear-and-tear of the imaging apparatus.

FIG. 1

**Description**

FIELD OF THE INVENTION

The invention relates to a system for imaging support, to a related method, to an imaging arrangement, to a computer program element, and to a computer readable medium.

BACKGROUND OF THE INVENTION

Machine learning ("ML") may be used in medical practice to assess image quality ("IQ") of medial imagery, such as mammograms or chest x-rays, etc. Such ML based quality assessment may also be used in other x-ray studies (e.g., in orthopedics), or indeed in other imaging modalities, such as volumetric imaging studies (e.g., CT, MR, ultrasound).
Typically, the ML-powered quality assessment inspects one or more aspects of image quality. For example, image quality may be defined in terms of: i) field of view ("FOV"): for example, "*is the entire region of interest captured on the image* ?", ii) patient posture, for example, "*is the patient standing straight* ?", iii) acquisition time (such as inhalation/exhalation), for example, "*is the patient inhaling properly* ?", iv) imaging parameter settings (such as radiation quality), for example, "*is the image under- or overexposed* ?", v) motion artefacts, for example, "*did the patient move during acquisition ?*", etc.
The image quality aspect or metric of interest may depend on the study type and/or imaging modality used.
When a radiologist receives a low-quality image study, he or she aims to answer the clinical question at hand to the best of his or her ability. However, if the image is non-diagnostic, the patient is invited for another imaging session, which is inconvenient and stressful for the patient, whilst also generating otherwise avoidable imaging volume, putting strain on imaging equipment, and exposing patient and/or staff to avoidable X-ray dosage for example.
ML-based quality assessment can help prevent low-quality studies from entering the radiological evaluation process. ML-based IQ assessment substantially immediately after acquisition may help alert the operator of the imaging equipment to any potential quality issues. A decision may then need to be taken on whether to acquire a new image whilst the patient is still in the imaging room.

SUMMARY OF THE INVENTION

There may therefore be a need for improved imaging support.
An object of the present invention is achieved by the subject matter of the independent claims, with further embodiments incorporated in the dependent claims. It should be noted that the following described aspect of the invention equally applies to the related method, the imaging arrangement, to the computer program element, and to the computer readable medium.
According to a first aspect of the invention there is provided a system for, preferably medical, imaging support, comprising:

an input interface for receiving an input image of a patient acquired by an imaging apparatus, the said input image previously assessed by an image quality assessment module and awarded a first image quality, IQ, score; and
an imaging triaging logic, configured to retrieve, or at attempts to retrieve, from an image database of prior assessed images, a corresponding image that corresponds to the said input image, the said corresponding image being a prior image of said patient or of a similar patient, and the imaging triaging logic to provide a decision, based on the first IQ score and a second IQ score of the corresponding image, if any, whether or not to acquire a new image of the patient by the imaging apparatus.

In embodiments, the system comprises a visualizer component to indicate i) whether or not such a corresponding image has been found, and/or ii) the IQ second score of said corresponding image, if found, and/or iii) an indication of the decision by the imaging triaging logic.
In embodiments, the visualizer operates to visualize, on a display device, the said corresponding image.
In embodiments, the system comprises a user interface ("UI"), configured to receive a user request for the imaging apparatus to acquire the said new image. Preferably, but not necessarily, the UI is integrated into a graphical UI ("GUI"). The GUI may include a graphics display for visualizing the corresponding and/or current image.
In embodiments, if no such corresponding image can be found, or if the second IQ score of the corresponding image does not exceed the first score, the imaging triaging logic (also referred to herein simply as "*the logic*" or "*the triaging logic*") to decide that such a new image is to be acquired, and to i) issue a control signal to the imaging apparatus to effect a re-acquisition of the new image of the patient or ii) effect indicting to the user of the imaging apparatus a recommendation to acquire the new image.

In embodiments, the imaging triaging logic is to retrieve the corresponding image from within a predefined prior time interval.

In embodiments, the time interval is dependent on any or more of: patient status, imaging protocol. The patient status may be one as awarded by a medical facility (clinic), and may include status of in-patient or out-patent, and the type of study performed or certain medical actions in relation to patient within the time interval, such as chest X-ray study, mammography study, etc.

In embodiments, the retrieved corresponding image is one having a region of interest at least overlapping with the region of interest in the current image.

In embodiments, the decision by the imaging triaging logic is one of not to acquire the new image, if the second score is larger than the first score and/or wherein the second score is larger than a pre-defined minimal IQ score.

In embodiments, the first and/or second IQ score is localizable in the respective image. In embodiments, the decision by the logic is further based on a clinical indication in respect of the current image and/or of the prior image. The clinical indication or imaging purpose represent the medical reason why the current and/or prior image was acquired. The clinical indication may be explicitly provided by user, or may be inferred by the logic from i) imaging protocol used, ii) medical records, iii) meta-data, etc. ML or search tools may be used for inferring this information. The clinical indication may be used by the imaging triaging logic to find the corresponding image, that relates to the same or similar ROI as the current image. This information allows guiding the search by logic in the prior imagery database.

The proposed system may allow harnessing, for imaging decision support, prior imagery as may held in databases. The prior imagery may be of the same or similar patient as the current patient. The proposed system allows retrieving such prior images that may serve, for the current IQ-deficient image, as a substitute image of sufficient IQ for the current region of interest ("ROI"). A retake may thus be avoided. That is, even though a current image study might be of low quality, a re-take may not be necessary if the patient had a similar study done not so long ago, that was of acceptable quality. The proposed system and method may thus support extensions of current radiological quality assessment in the clinical workflow. A prior image corresponds to the current image if it relates the same region of interest as the current image does.

The proposed system may be used to assist technologists in deciding on whether a re-take is necessary. The system may be implemented on high performance computing equipment with prompt turnaround of decision support information in near real time so that the user can act on this information whilst the current patient is still in the imaging room. The system can quickly correlate a current low-quality image with relevant prior image(s) as may be held in databases for the current patient, or for patients similar to the current patient.

The risk of unnecessary image retakes can be reduced, thus saving time, X-ray dose, and reducing wear-and-tear of the imaging apparatus.

In some embodiment leverage on the fact that some (prior) ML based quality assessments are regional, i.e., they pertain only to a portion/region (subset) of a prior image in a prior study, whereas other ML assessments are global, i.e., they relate to the whole of the image or study. For instance, FOV-issues are typically regional, while radiation issues are typically global. In particular, for local image quality assessments, some prior image(s) may be of insufficient IQ in respect of some region, but may be of sufficient IQ for other regions, currently of interest for the current image, but may not have been of interest when the prior image was taken.

In another aspect there is provided a computer-implemented method of imaging support, comprising:

receiving an input image of a patient acquired by an imaging apparatus, the said input image previously assessed by an image quality assessment module and awarded a first IQ score;
retrieving from an image database of prior assessed images, a corresponding image that corresponds to the said input image, the said corresponding image being a prior image of said patient or of a similar patient; and
deciding, based on the IQ score and a second IQ score of the corresponding image, if any, whether or not to acquire a new image of the patient by the imaging apparatus.

In another aspect there is provided an imaging arrangement, comprising the system, and the imaging apparatus, the imaging apparatus being preferably of the medical X-ray type.

In another aspect there is provided a computer program element, which, when being executed by at least one processing unit, is adapted to cause the processing unit to perform the method as per any one of the above-mentioned embodiments.

In another aspect still, there is provided a computer readable medium having stored thereon the program element.

*"use/operator"* relates to a person, such as medical personnel or other, operating the imaging apparatus or overseeing the imaging procedure. In other words, the user is in general not the patient.

*"re-take"* may refer to either a partial or whole re-take of the image study. A partial re-take incudes repeat imaging of the anatomical region of interest, not optimally imaged as per the current image.

BRIEF DESCRIPTION OF THE DRAWINGS

Exemplary embodiments of the invention will now be described with reference to the following drawings, which are not scale, wherein:

Fig. 1 shows a schematic block diagram of an imaging arrangement including a computerized imaging support system for support a user; and
Fig. 2 shows a flow chart of computer-implemented method for supporting imaging.

DETAILED DESCRIPTION OF EMBODIMENTS

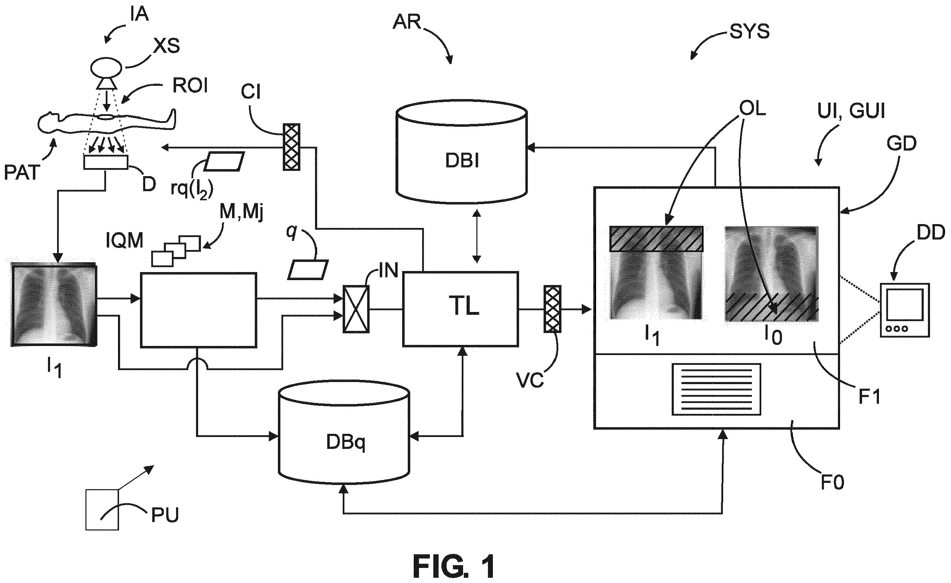

Reference is now made to the bock diagram in Fig. 1. This represents components of an imaging arrangement AR as envisaged herein.

The imaging arrangement AR may include an imaging apparatus IA configured to acquire imagery of a patient PAT in an imaging session. The imaging session may take place in an imaging room in a medical facility for example. The arrangement AR may further include a computer implemented system SYS configured to process the imagery.

The system SYS may receive the imagery directly at the point of acquisition ("in-room") from the imaging apparatus AI. Alternatively, although less preferred herein, the imagery is first stored in a memory, and is then later (for example after completion of the imaging session) retrieved therefrom to be processed by the system SYS. However it is in particular the in-room use that is preferred herein, with rapid processing turnaround: the imagery is received and processed by system SYS whilst the patient is still in the imaging room, in particular whilst the patient is still at the imaging apparatus IA. More specifically still, and in some scenarios envisaged herein, patient PAT may still have a region of interest (ROI) located in an examination region of the imaging apparatus IA ("imager") when the current image is processed by the system SYS.

The system SYS is configured to support a user (such as medical personnel, radiographer, technologist etc) in operating the imaging apparatus IA. Very broadly, and as will be explored more fully below, the system SYS processes current imagery and produces a recommendation on whether a retake is called far. For an IQ-deficient current image, a prior image of better (sufficient) IQ may be retrieved by system SYS as a substitute for the current image. The imagery acquired by the imager IA may be forwarded to the system SYS for processing via a wireless or wired communication network. The system SYS may be integrated into an operating console of the imager IA, or may be integrated in a computing environment, such as workstation, associated and communicatively coupled to the imager IA.

A range of imaging equipment IA is envisaged herein, including those configured for emission or transmission imaging. Preferably, imager IA is configured for transmission imaging, such as x-ray imaging. Radiographic or tomographic imaging are both envisaged herein. In radiography, projection image(s) is/are acquired, whilst in tomographic imaging, the desired imagery is cross-sectional imagery, computationally reconstructed - by using a reconstruction algorithm - from a set of projection images acquired from different directions around the ROI. In the following, no distinction will be made between projection imagery or reconstructed cross-sectional imagery, and both will be simply referred to herein as (acquired) imagery/image/images.

The imagery may be displayed on a display device DD. In general, in x-ray imaging, the imager IA includes an x-ray source XS (such as an X-ray tube) and an X-ray sensitive detector D, arranged opposite the x-ray source so that the examination region is formed in between the two. The patient PAT, in particular the region of interest, is positioned in the examination region of the imager IA. The patient may lie on an examination table during the imaging session. The table with the patient on it may be introduced into a bore (an example of the said examination region) of the imager such as in CT imaging. However, this is not a necessity herein as the patient may also sit or stand in the examination region as required.

During image acquisition in the imaging session, the x-ray source XS is energized to produce an x-ray beam of suitable energy, depending on the imaging objective/purpose. The x-ray beam passes through the patient, preferably at the ROI. The X-radiation interacts with patient tissue. The interaction causes modified radiation. The modified radiation is detected by the x-ray sensitive detector D. Acquisition circuitry including an analogue to digital conversion unit DAQ (not shown) converts detected radiation intensities into digital image(s) comprising image values stored in rows and columns for example. One such image $I_1$ acquired in an on-going imaging session is shown in Fig. 1. More than one image may be acquired as per an imaging protocol, with the beam suitably aligned and having a prescribed energy (range).

The imager AI may or may not include a collimator (not shown) to collimate the X-ray beam accordingly, preferably tightly around the region of interest. The region of interest may be formed by an organ of interest or part thereof, a group of organs, tissue or part(s) of an anatomy of interest.

In general, and ideally, the (physical) region of interest may correlate to a certain image portion in the acquired imagery. The physical region of interest thus corresponds to a sub-set of pixels or voxels in the acquired image. Imagery is made up from image values addressable in a matrix structure of rows and columns at different pixel or voxel positions.

For simplicity, we will be using herein as a common designation "pixels" to refer to image locations in both, projection or reconstructed cross-sectional imagery. The acquired image may be an image volume in 3D or 4D (a time series of 3D image), or may be 2D projection or reconstructed cross-sectional image, either still or as part of a stream for frames (video feed) such as in fluoroscopic imaging, also envisaged herein. For simplicity, we will refer herein to the (image-)ROI as a sub-set of pixels of a given image $I_1$, it being understood that there is in general a correspondence between the anatomical, physical ROI ("patient"-ROI) of the patient anatomy, and its representation in the acquired imagery as a sub-set of pixels, the image-ROI. This correspondence exists when the FOV was properly adjusted relative to the physical region of interest. There may not be a full one-to-one correspondence if parts of the image-ROI is cut off as result of improper FOV alignment. This situation may attract a lower FOV IQ score, on which more below.

Whilst main reference will be made herein to x-ray imaging using ionizing radiation, as said other imaging modalities such as MRI or others still, are not excluded herein. However, it is in particular with x-ray imaging using ionizing radiation that there is a trade-off situation. On the one hand, the exposure to x-ray during image acquisition is as such harmful as genetic material in the affected tissue cells may be harmed, potentially causing cancer. It is therefore a desire to reduce radiation dosage exposure and to not take more images than is medically necessary. On the other hand, and in an opposing sense, the images should be diagnostic, acquired at sufficient dose, so that the region of interest can be represented clearly and at sufficient/good IQ, such as at high contrast and/or with low noise, etc. In addition, alignment is of import, a geometrical requirement, in that the ROI should be preferably included as a whole into the current FOV of the imager. The image-ROI should be wholly within the image FOV, rather than be cut off at the edges of the image field of view. Imaging parameters need be adjusted, manually, semi-automatically, or automatically, so that the patient ROI is correctly positioned in the examination region to ensure that the FOV of the current image includes preferably all the image ROI as a sub-set of pixels.

For various reasons, such as skill/training level of medical user operating the imager, or uncooperative patients (patient motion), etc, it may happen that the acquired imagery turns out non-diagnostic/of insufficient image quality. This is the case for example when the said region of interest is not entirely included in the current field of view as per the current imaging parameters, and/or when patient positioning/posture is not correct. Another example of poor IQ or non-diagnostic imagery is when image contrast is too low or noise level is too high. This may be caused by under-exposure for example, where voltage/amperage of the x-ray source XS was incorrectly adjusted by the user.

In such situations of non-diagnostic/low quality IQ, the question frequently arises whether a re-take is necessary, where a new image 12 is acquired. Such a re-take may be necessary, in case of incorrect protocol and patient position for example. Re-takes should be avoided as much as possible, for example to reduce cost, to increase patient throughput, and to reduce technical wear-and-tear on the imaging equipment itself. For example, in x-ray imaging, the x-ray tube XS includes a cathode disc which is subjected during acquisition to extremely high temperatures/temperature gradients. Unnecessary re-takes may thus prematurely wear out the x-ray's cathode disc which may call for expensive maintenance work, service personnel call-out and associated down time. It is also in particular with x-ray imaging using ionizing radiation that patient/staff's health may be at risk, as too liberal a view on re-takes exposes patient and clinical users to unnecessarily high x-ray dosage.

On the other hand, it is essential that the image is diagnostic in the sense that its image quality is according to protocol and meets requirements to ensure correct diagnosis or therapy based on the information in the imagery. It is of no use for example, if retaking is avoided, and to then later find that a wrong clinical decision was made due to poor image quality.

There is thus a tension between the decision to avoid retakes, and the desire for diagnostic/high quality imagery. In order to address this tension, and to alleviate the situation, the proposed computerized system SYS is configured for imaging support. It supports the user of the imaging equipment in their decision on image re-take. Broadly, the proposed imaging support system SYS harnesses historic imagery of the same, currently imaged patient, or of physically similar patients, that may be used as substitute imagery at higher IQ. It is this substitute imagery which may be used instead of the currently acquired image, or in conjunction therewith, for diagnostic purposes, such as in a radiological image review session by a radiologist, or for other purposes. If a suitable substitute image $I_0$ can be found in an image database DBI, a re-take of a new image $I_2$ can be avoided. The image database DBI may for example be implemented as a PACS (Picture Archiving and Communication System), a hospital information system (HIS), or other image repository.

In more detail, the proposed system SYS may include an imaging triaging logic TL. The logic TL retrieves, if extant, suitable substitute imagery from one or more databases DBI that hold historic patient imagery. The substitute imagery, if any, is identified by the logic TL among historic prior imagery $I_0$ as held in database DBI and may be retrieved therefrom through a suitable retrieval interface. The logic is programmed and arranged to use suitable conditions as will be explored below in more detail at (1)-(3). The substitute imagery, if found, may be rendered for view in a graphics display GD on display device DD by a visualizer component VC. The graphics display GD may be displayed on display device DD in the examination room, preferably immediately/shortly after acquisition of the current image $I_1$.

The graphics display GD may include two fields F1, F0. Field F1 may include a visualization of the retrieved substitute image $I_0$, possibly visualized concurrently with the current image $I_1$. The optional, other field, the information field F0, may include an indication for a recommendation as to whether or not to effect a re-take. The indication may be in form of a message. Other channels may be used to generate an alert signal if a suitable prior substitute image $I_0$ is found or not found. For example, an (additional) visual or audio alert signal may be issued by controlling the device display and/or another transducer, such as acoustic transducer. The various modes of alerting the user on the outcome of the triaging logic's TL operation will be discussed in more detail below.

Preferably, the triaging logic TL of the imaging support system SYS is used in conjunction with one or more image quality module IQM. Image quality modules were described in the past. They may be implemented by using machine learning "(ML"). Such image quality modules IQM may be arranged as software module(s) that run on one or more computer systems, not necessarily on the computing system that implements the proposed imaging support system. The image quality module IQM assesses the current image $I_1$ for image quality IQ.

In more detail, the ML based image quality assessment module IQM may include one or more machine learning model M, Mj, previously trained on historic imagery. The model processes input image $I_1$ to produce an indication of an aspect of image quality as captured in the image. The output produced by the model M may be in the form of a scalar score, that is a number, for example in a predefined limited range *[a,b]* to facilitate comparison. For example, the range may be normalized (*a=0* and *b= 1*) to unity range, thus indicating image quality on the scale *[a,b]*. The score is a measure for image quality. The score may be thresholded. For example, a score of at least 50% may be required for the imagery to be considered diagnostic, but this depends on the exact manner in which the ML output is encoded. The score $q$ may be thresholded against a master score *Q*. The master score *Q* represents the minimum scoring for the image to be deemed of sufficient quality. Merely binary indications, such as a = *"sufficient"*, b= *"not sufficient"* are also envisaged. Thus, the ML model is in general arranged as a classifier. Different types of ML modelling approaches are envisaged herein for the machine learning model $M, M_j$, including neural network models, support vector machines, statistical techniques, and others. Regression type models are also envisaged.

The image quality score (referred to herein generally as *q*) awarded by the image quality module IQM may be for the image as a whole or may be regional. In such regional embodiments, the image quality module IQM not only outputs the said score $q$, but outputs in addition an indication of a sub-set of pixels in the image to which this score pertains. For example, co-ordinates of a bounding box may be output for the processed input imagery $I_1$, and the image quality module IQM relates to this part of the image. The IQ localization indicator *B,* such as said bounding box, may be displayed on the display device DD. The IQ localization indicator *B* may be superimposed on the current input image $I_1$ for example. As will be explained in more detail below, the IQ localization indicator *B* may be utilized herein for purposes other than displaying. Specifically, the IQ localization indicators *B* may be utilized herein to retrieve the substitute imagery $I_0$. Different scores may be computed for different portions of the very same image.

The image quality module IQM may include plural machine learning models Mj each trained for a different aspect of image quality. For example, one module M1 may be configured and trained to compute a score in respect of contrast, whilst another model M2 may score for collimation or image FOV conformance with image-ROI, that is, whether the region of interest is indeed fully in the field of view. Yet another model M3 may score for patient motion, whether the patient has been sufficiently motionless during acquisition, etc.

Thus, in embodiments a bank of plural trained machine learning models $M_j$ is used, each one trained for a specific type or aspect of image quality, and this is preferred herein. Each such model may produce a different IQ score $q_i$ that each relates to a different IQ aspect. Whilst one IQ aspect may be scored as sufficient by one model $M_j$, another IQ aspect may be scored by another model $M_k$ as insufficient. Also, the respective IQ localization indicators Bi may be different for some or each model $M_j$. The IQ localization indicators $B_i \subset I_1$ may be disjoint or may intersect to form overlap(s).

Thus, if such a bank of different machine learning modules is used, the respective scores $q_j$ can be treated separately. The imaging triaging logic TL may thus initiate different retrieval operations, for example at least one for each score $q_j$ that was found deficient. As a result, more than one substitute image may be returned by the logic TL for different scores that were found deficient. In particular, the logic may return multiple substitute images $I_{0j}$ for different scores. The logic TL may establish that there is no substitute imagery with respect to some scores $q_j$, but there may be substitute image(s) found for some other(s) score $q_k$.

As a variant, the multiple scores may be combined into an overall master score indicative of whether or not the image has passed the image quality check by all image quality models in respect of each image quality aspect measured by the respective machine learning models $M_j$. The master score $q = F(q_1, .., q_N)$ may be computed using a combiner function *F*, such as an average, weighted average, etc. The triaging logic TL may thus run a single retrieval operation to find a prior image $I_0$ that attracts a better such combined result.

In the alternative to the bank of models, a single model may be configured to produce all or some of such different imagery scores $q_j$ as a vector $q= (q_1, ... q_N)$. Plural such model Mj are hence not necessarily required herein.

In the following, for simplicity, we will confine explanations to a given, generic machine learning model M and its score $q$ (and its generic localization indictor *B* if applicable), with the understanding that everything that will be said herein is applicable to each of the machine learning modules Mj of the bank of such models.

As the image quality assessment by the image quality module IQM is preferably localized, the output (the assessment result *r*) of model M may be written as r= ($q$,B), where $q$ denotes the IQ assessment score, and *B* denotes the respective IQ localization indicator that indicates the image portion (subset) in image (such as image $I_1$) to which the image quality score pertains. As illustrated in Fig. 1, the IQ localization indicator B may be displayed as an overlay graphics OL in the graphics display GD when displaying the current $I_1$ and/or the proposed substitute image $I_0$.

Turning now in more detail to operation of the triaging logic TL, this determines whether the current image $I_1$ is of sufficient quality and may produce a signal or indication that corresponds to the result of this determination. The said result is based on the score $q$ received from the image quality module IQM. Alternatively, the result is determined at the image quality module IQM, and a message is forwarded to the triaging logic TL to inform the triaging logic TL about the IQ assessment result.

The triaging logic TL may then determine and effect a suitable action. One action is one of the advisory type, where the logic TL instructs the graphics display generator DGD to generate and display the graphics display to inform the user about the result on IQ. Advisory type action is optional, but preferred herein to ensure the user remains in the "loop", but could be left out, for example in an autonomous imaging setup. The result on image quality is preferably logged in either case. Another action type determined and effected by the logic TL is of the remedial type, which includes attempting to retrieve a fitting substitute image $I_0$, if the image quality of the current image is determined as not sufficient.

Turning now first to the advisory type action as effected by the logic TL, if the IQ is determined sufficient, this fact may or may not be indicated explicitly. For example, if required, a suitable all clear message and/or other signal, such as indicator widget in a green color or other encoding, may be included in the graphics display GD. However, if the image quality is not sufficient, an alert message and/or other signal may be generated. This alert signal may comprise a message which may be included in the displayed graphics display GD. For example, a color-coded alert widget of preferably sufficient conspicuity, such as in a red color, may be displayed. In addition or instead, the information field F0 is made to include a certain text, indicating that there is a quality issue with the current image $I_1$. The fails quality score may be displayed. Other alert signals, such as an audio signal may be sounded out by the logic TL controlling through a suitable interface an audio transducer.

The user may then review the display information or act on the alert signal, preferably whilst the patent PAT is still in the intended position at the imaging apparatus and in the imaging room. The user may or may not agree with the determination that the current image is of insufficient IQ. The displayed information may further indicate the one or more remedial action: i) retake or ii) attempt retrieving substitute image. The user may request retake or may request retrieval of the substitute image $I_0$. The remedial action options may be indicated graphically, textually, or otherwise in the information field F0. Interactive GUI widget(s) may be used, integrated in the graphics display. The widget(s) respond(s) to user input (via pointer tool or touch screen action) to allow user selection of the remedial action. Any other user interface input arrangement may be used instead. The proposed setup is not confined to GUIs, although this is preferred as it facilitates seamless and quick operation.

Alternatively, if the input image $I_1$ is determined to be of insufficient quality $I_1$, the remedial action of substitute image retrieval is requested automatically, and no (further) user input is required. The fact that the retrieval operation is underway may still be indicated in information field F0, by displaying a textual note to this effect, and/or by displaying a preferably dynamically updated progression bar widget, or similar.

Referring now in yet more detail to the remedial action of substitute image retrieval in case the input image $I_1$ is not of sufficient quality, the triaging logic TL accesses one or more image databases DBI through suitable database retrieval interfaces, to find the substitute image(s) $I_0$. The retrieval operation may be user requested or auto-triggered based on IQ determination. If such a (proposed) substitute image $I_0$ is found, this fact may be displayed on the graphic display GD, for example in information field F0. Alternatively, or in addition, the substitute image itself may be displayed in field F1, either alongside the current image $I_1$. or instead the current image $I_1$. The information field F0 may include an indication and/or a quantification of the extent or degree to which the retrieved image can substitute the current image. If no prior substitute image $I_0$ is found, this fact may be indicated as an alert signal or message, possibly with an indication that a re-take may be necessary. A GUI widget integrated in the graphics display GD may be provided to allow the user requesting retake, or, less preferable but envisaged in particular in autonomous imaging settings, the retake is auto-triggered by the event of no substitute image found.

Conditions used by the triaging logic TL to determine that a suitable prior image can be identified in the retrieval operation as a substitute image are now explained in more detail. Operation of triaging logic may be distinguished, based on the configuration of IQ measure *q*. Specifically, the IQ measure q may be computed by IQ module IQM to measure to what extent the ROI is within the FOV. Such an IQ score may be referred to herein as a "FOV-score". Otherwise, if the IQ score q is configured to measure other IQ aspects than ROV versus FOV, such as contrast, etc, the measure may be referred to herein as a non-FOV score.

In one embodiment, and turning now first to the case where the score q is a FOV-score, the triaging logic TL determines a prior image $I_0$ in an image database DBI to be a substitute image if:

$$q_0 > q_1 \qquad\qquad (1)$$

with $q_0$ the IQ quality of current image, and $q_1$ the IQ of the prior image $I_0$. That is, if a prior image $I_0$ is retrievable as a substitute image, its FOV includes a larger portion of the ROI than does the current image $I_1$.

Turning now to the case where measure q is a non-FOV-measure, in this case the triaging logic TL uses the concept of ROI overlap. Specifically, the triaging logic TL determines a prior image $I_0$ in an image database DBI to be a substitute image if its image region of interest, referred to herein as ROI_0, overlaps with the region of interest ROI_1 of the current image, and has an image quality score greater than the current image or its ROI_1. This condition may be framed as:

$$(q_0 > q_1) \wedge ( \mathrm{ROI\_0} \cap \mathrm{ROI\_1} \neq \varnothing ) \qquad\qquad (2)$$

For example, this may include the case where the prior ROI-0 equals (is coterminous), or is greater than the current image region of interest ROI_1. Thus, preferably, the triaging logic as envisaged herein, in particular for the case of non-FOV score q, may be configured to find a substitute image which is one where its ROI_0 has sufficient IQ, and at least fully includes the current region of interest ROI_1.

In general however, the two ROIs may not be so related as $\mathrm{ROI\_0} \supseteq \mathrm{ROI\_1}$, but there may merely be an overlap defined by the intersection of the two ROIs. The greater the overlap, the more likely it is that this image $I_0$, given that it has sufficient IQ, may still serve as a substitute for the current image $I_0$.. The logic TL may restrict the search, so that only historic/prior imagery is considered that have a certain minimum overlap of say at least $p$% (eg, 5%) with the current ROI_1 of the current image. The triaging logic TL may hence operate a thresholding scheme to retrieve possible substitute imagery.

When there is no overlap, that is, when (1) or (2) is not fulfilled for all priors, then the triaging logic returns a signal that the retrieval operation failed. The fail signal may be indicated on the graphics display, optionally with an indication that the retake should be considered. Alternatively, the retake is initiated automatically, optionally with the fail signal so indicated.

Thus, as proposed herein, the imaging support system allows substantially decreasing the risk for unnecessary re-takes as the triaging logic TL can be configured for a broad query, more likely to yield a hit, by adjusting the minimum overlap $p$.

The determination of the overlap may be done in plural ways, all envisaged herein in embodiments. For example, if the regions are encoded by a collection of pixels as per the respective locator $B$ (bounding box for example), the overlap may be quantified by computing the size of the area of overlap. For example, the size of the area of overlap may be computed relative to the size of the combined areas of two pixel collections, that is, of the current image-ROI ROI_1 and of the image-ROI in the prior image ROI_0. The Jaccard index or Dice score may be used to quantify the overlap.

In embodiments, the current and prior images $I_0$ are registered before the overlap area is determined. In yet another embodiment, the anatomies as per the ROIs are detected on the images $I_0, I_1$, and mapped onto a normal space before the overlap area is determined.

If the regions are implicitly defined by codes rather than by pixel subsets (as per locators B), the ROIs may be related by identity matching over the codes. For example, if the "north side" of a chest x-ray is IQ compromised on the current image $I_1$, and the "west side" of a prior image $I_0$ is compromised, then these would not be considered as a "match" since there is no overlap as in this example "north side" $\neq$ "west side". In a refined embodiment, the codes may be hierarchically ordered in a tree structure, e.g., "north-east corner" is-a-part-of "north side", where the tree can be of depth 2 or more. In this embodiment, simple identity matching would be replaced by checking if one code is hierarchically subordinate to the other.

The operation of logic TL described above in embodiments is one based on improving IQ. However, both current and substitute $I_0$ may still fall below the master IQ score Q. This fact may be indicated to the user in a message on the graphics display. The above described conditions (1), (2) may be complemented by an additional condition (3), mandating the prior IQ score q0 must further at least met the master score Q:

$$q0 \geq Q \qquad\qquad (3)$$

Thus, with (1) or (2) complemented by (3), the retrieved prior image $I_0$ is considered a substitute image only if its IQ score is at least $Q$. Leaving condition (3) out may broaden the search and the likelihood for a no retake may be increased as in some cases a mere improvement over the current score q1 may provide all the information the clinical user was after.

As mentioned above, the graphics display GD generated based on the output of the triaging logic TL may represent the localization indicators *B* as overlays OL superimposed on the respective imagery to indicate the respective regions of interest. Instead of or in addition to said overlays, the associated quality score(s) q may also be indicated. Output of the triaging logic may further include an indication on whether or not a substitute image has been found. The extent of the overlap of its ROI_0 with the current ROI_1 match may be indicated, such as in percentage terms *p*.

It may be the case that the historic prior imagery as stored in the database DBI had IQ scores awarded only for single image region that was the then region of interest ROI_0. In such cases, the triaging logic TL may be configured to define additional regions of interest that that may be compared with the current region of interest for the current imagery as per (1),(2). In this embodiment, the triaging logic may be configured to interact with the IQM module and instruct same to post-compute the respective scores for the said other image portions in the historic imagery. However, this proposed post-computing of image quality scores for historic image data may not always be feasible.

To this end, another embodiment is envisaged, where the triaging logic optionally registers the current image $I_1$ one-by-one with each of a suitable set of candidate prior images $I_{0j}$. The registration is optional as the prior images may already be at least in approximate alignment with the current image $I_1$. The imaging triaging logic TL effects that the current ROI_1 is mapped into each of the so registered prior images to so define or "import" corresponding prior regions of interest ROI_0$_j$. The bounding box $B_1$ may be used to define corresponding ROIs in the candidate images. The IQM module is then instructed by imaging triaging logic TL to post-compute IQ scores for the so imported new regions of interest ROI_0$_j$. The one or more of the candidate images that yield a score $q_{0j}$ greater than the current score $q_1$ for ROI_1 may then be output as potential substitute image. The single candidate image that attracted the highest score in the imported ROIs may be output as the substitute image. The candidate set of images may be defined by a pre-search for prior images for the same patient, or for patients that have similar bio-characteristics. This embodiment based on imported ROIs may consume considerable computing power and may require the candidate set to be of modest size. However, if computing power is of no concern, for example if multicore processors are available and/or the task can be reasonably parallelized, the whole database may be so searched based on post-computed IQs for imported ROIs.

Post-computing ROIs in priors as indicated above may not always be feasible. In such cases, embodiments are envisaged where the logic TFL searches the databases for priors with ROIs pre-defined corresponding to the current ROI.

Specifically, the retrieval operation by logic TL may determine which type of regions of interest there are in the prior images and in the current image. Specifically, it is determined by triaging logic TL to which anatomy the ROIs relate to. For example, if the current ROI_1 in current image $I_1$ is for a shinbone (tibia) in the right leg, the triaging logic TL may search for other shinbone ROIs in the prior images. The anatomy/tissue or anatomical feature to which a ROI refers to may be called herein the type of ROI/ROI type. The type of region of interest ROI_1 for the current image $I_1$ may be explicitly defined by user input. The system SYS may provide for a suitable user interface such as a GUI or otherwise, optionally part of the graphics display GD, for the user to specify the ROI type. Alternatively, ROI type may be inferred automatically from specifications or metadata, in particular form the imaging protocol used for current image $I_1$. An indication of the respective regions of interest in the current or prior images may be derived from header data for example. A useful image format in this regard is the DICOM standard, envisaged herein in embodiments. DICOM (Digital Imaging and Communications in Medicine) is an international and widely used format for storing and sharing medical image data.

Alternatively or in addition to analysing protocol data, the logic TL may be configured to infer the ROIs by analysing clinical indications or specification of the imaging purpose for which the current and prior images were acquired. For example, a purpose statement or clinical indication such as: "*suspected fracture of the seventh rib on the right hand side*", may be sufficient to infer the region of interest, in this case the seventh rib on the right hand side. This type of metadata/ contextual data on clinical indication or imaging purpose may be obtained from medical reports and records associated with the historic imagery or may be otherwise inferred from other contextual image or non-image data. The clinical indication/purpose for the imaging may be indicated by a code in header data of the historic imagery, such as may be the case for images of the above referenced DICOM type.

The imaging triaging logic TL may include a natural language processing (NLP) pipeline to analyse textual information in said patient records or other contextual or meta-data to determine imaging purpose or clinical indication for prior images, to so derive the type of region of interest. The NLP may be based on machine learning. In addition or instead of such NLP processing, the triaging logic TL may include a machine learning component configured and trained to infer from contextual /metadata, such as the imaging parameters used, the region of interest in the historic and current imagery. Neural network type models, such as CNNs, may be used. The model may be trained for example on expert-labelled training data based on historic imagery and their associated records.

As another embodiment, if the score q awarded to the prior imagery $I_0$ is localized, the associated localizer B may be used as an indicator for the type of ROI.

In any one of the above-described embodiments, if there is more than one such prior image $I_{0j}$ that may serve as a substitute, these may be displayed in the graphics display GD, either concurrently, or sequentially, one by one or in groups. For example, the graphics display may include a GUI widget for the user to scroll through the set of potential substitute imagery $I_0$.

If suitable prior imagery $I_0$ was found by logic TL or by the search conducted on behalf of logic TL, this may be indicated to the user in the information field F0, textually and/or graphically. Automated embodiments are also envisaged, in particular for autonomous imaging settings, where a suitable control interface CI is provided through which the triaging agent TL can automatically put through a request $rq(I_2)$ for a re-take of the new image $I_2$, in case no substitute image $I_0$ could be found. Alternatively, it is the user who puts through this request $rq(I_2)$ via an user interface.

The above-described retrieval operations by the triaging logic TL may be constrained by the type of imagery and/or patient data. For example, the search will be confined to prior imagery of the very same patient or to imagery of patients who have sufficiently similar bio-characteristics as the current patient. In addition, the search may be constrained based on the types of regions of interest that match the type of current region of interest in respect of image quality. A further search constraint related to the overlap and IQ criteria as defined in queries (1) and/or (2).

However, the search operation can be further constrained usefully, for example time constraints may be used. Specifically, a time period may be defined to restrict the search. In embodiments, the time interval in which prior imagery is considered may depend on the study type. This may be established by means of a look up table, that may specify for example a time period of 2 days for chest x-ray, but may allow 90 days for a mammogram, etc.

In yet another embodiment, the time period is dependent on the study type. The study type may be indicated by a set flag such as *"normal", "stat",* etc. The flag may indicate a prioritization status in a reading queue of a radiologist. For example, a set flag *"stat"* may indicate a higher urgency than flag *"normal"*.

In yet another embodiment, the time constraints may be modulated by changing patient characteristics or attributes. For instance, if the patient is an in-patient at the time of the current study, then the time period may be restricted to extend back in time only for as long as the patient was still an in-patient. The time interval is restricted to exclude time intervals where the patient was an out-patient for example. For example, the search for prior imagery does extend prior to a time when the first non-in-patient study was done. In yet another embodiment, such search stops (time points prior to which the search will not be extended) may be defined by detecting time points when a change in the Electronic Medical Record occurred, for example new entries that indicate new medication, new lab values outside of normal ranges, new problems on problem list, etc.

As shown in Fig. 1, the prior imagery may be held in database(s) DBI, while the quality assessments Q of the said prior imagery may be held in a separate database DBQ as computed in prior IQ assessments.

In more detail, an IQ score database DBq may record outputs (score q or, if applicable, localized score $(q,B)$) of the IQM module from a radiological quality assessment, preferably along with information uniquely specifying the image and the patient. This unique identification may be implemented by using accession number, study type and Medical Record Number (MRN). Such data items may be stored in meta-data of DICOM imagery. The database can be queried for a patient's most recent study of a certain type (e.g., chest x-ray) using a standard Application Programming Interface (API). The image, as opposed to its score, may be stored in PACS or other image repository/database. The triaging logic TL may use suitable interfaces (API - application program interface) to access and locate prior imagery in the PACS. Specifically, a query can be devised that enables automated retrieval of a patient's image(s) of a certain study type (for example, chest x-ray) within a certain time interval (for example, within the last month). Instead, or in addition, patient images for patients having similar bio-characteristics and/or medical history as the current patient may be accessed when searching for prior imagery.

The above-mentioned separate storing of prior imagery and their historic IQ assessments is not a requirement herein, and both may be stored on the same database, each prior image being associated with its historic quality assessment score.

Whether stored on the same database or on different databases, suitable identifiers are required for the imagery and the historic quality assessment so as to unambiguously retrieve and associate the imagery with their associated quality assessment. Alternatively, the quality assessment results may be stored in header data of the respective image.

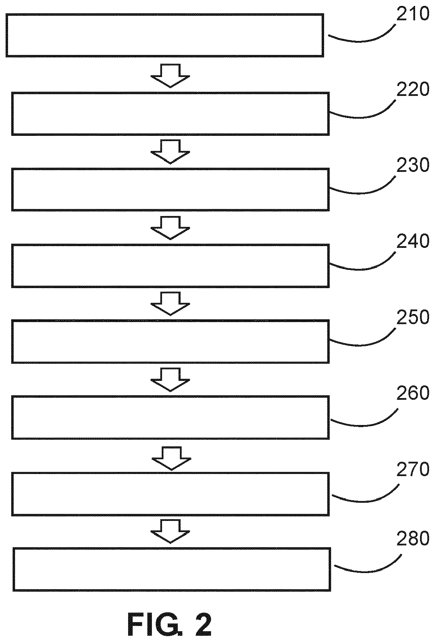

It will be appreciated that if the quality assessment is not localized but is global, the above principles can still be applied, in which case it may be sufficient to find prior images whose ROI type matches the current ROI type, and apply queries (1) and/or (2) to establish the suitable substitute image $I_0$. Reference is now made to the flow chart of Fig. 2, which illustrates steps of a computer-implemented method that may be used to implement the above-described system. However, it will be understood that the method described below is not necessarily confined to the system described above, and may be construed as a teaching in its own right.

The proposed imaging support method provides user support on imaging decision. The method facilitates a decision on whether or not to acquire a new image $I_2$ in a retake, after an initial image $I_1$ was acquired. The method helps avoiding unnecessary retakes. A likelihood of additional X-ray dosage exposure or premature wear-and-tear on imaging equipment may be reduced thanks to the method.

At step S210 the current image $I_1$ is acquired, for example by an X-ray based imaging apparatus. Other imaging modalities, such as US, MRI, PET, SPECT, etc are not excluded herein instead of X-ray or in addition thereto.

At an optional step S220, the current image is forwarded through a communication network for IQ assessment. Step S220 is optional in that the computations for performing the quality assessment may be done on site, for example in the examination room, by a local computing system integrated with the imaging system

At step S230, the current image is assessed by a computer-implemented IQ assessment module. An image quality score is computed. The module may be based on trained machine learning model configured for regression or classification. A neural network type model, preferably of the convolutional type, may be used, but other ML models such as SVM (support vector machine), decision trees, etc. are not excluded herein. The IQ score computed may be localized or global. In case it is localized, the computed assessment result includes not only the IQ score $q$ (thresholded or not), but further includes a specification $B$ of a subset in the acquired image $I_1$, to define the set of pixels to which the assessment score pertains. The assessment result computed by IQ module M may thus be written as $M(I_1) = (q,B)$.

Preferably, the image IQ score/ classification result $(q,B)$ is computed at step S230 immediately after the image acquisition at step S210. Preferably, the IQ assessment is completed whilst the patient is still in the examination room, specifically whilst the patient or a part (the ROI) of the patent is still in the examination region of the imaging system. For example, the IQ assessment is available whilst the patient is still lying on an examination table or is otherwise at least partly located in the examination region, with the ROI still positioned in the examination region, or the patient is otherwise still in or at the imaging equipment. The examination region may be the space defined between an x-ray source and an X-ray-detector of the imaging equipment.

At step S240, if the computed IQ score is found to indicate that the IQ of the current image in respect of a given image-ROI is not sufficient, a search/retrieval operation S250 is done in historic prior imagery of the same or similar patient.

Step S240 may optionally include indicating by text, video or graphically that a IQ issue was detected and that the system intends to retrieve prior imagery to find a suitable substitute image.

The search/retrieval operation S250 is configured to find prior imagery that includes, at least in parts, an image-ROI that corresponds to the image-ROI of the current image and has greater (or at least or the same) quality score q as determined at step S230. The correspondence of the said image ROIs may be quantified in terms of overlap as sets of pixels. Specifically, the ROI-wise correspondence may be measured in terms of the overlap between the current image-ROI and the image-ROI in the prior image, in particular for non-FOV scores q. The condition (1) or (2) may be used in the retrieval operation S250, optionally in combination with condition (3).

If such as prior image is found at step S250, this prior image $I_0$ is considered a substitute image for current image $I_1$.

The search/retrieval operation at S250 may be restricted to prior imagery having an image-ROI of the same type as that of the current image, and/or at least having a certain minimal overlap $p$.

At an optional step S260, the substitute image $I_0$, if found, may be displayed on a display device in a suitable graphics display, with or without the current image.

As an option, in step S270, if a prior image is found that scan serve as a substitute, a decision is issued that no retake is required. The decision may be modulated into a signal for the user as a graphical signal, a textual message, a video signal, or as an audio signal, etc.

At step S280, if no substitute image could be so found, a decision/recommendation that a retake may be required is issued. The decision may be displayed, or may be otherwise modulated into a user perceptible signal. Alternatively, or in addition, the re-take is initiated automatically.

The search step S250 may include comparing the region of interest in prior images and the region of interest in the current image to establish the overlap. Only prior imagery with image qualities for the corresponding region of interest of at least the quality of current image as established at step S230 may be considered herein as per condition (1) or (2) above, optionally in conjunction with (3).

Time constraints and/or other constraints in respect of the patient's medical history, etc may be included when searching for the imagery at step S250. For example, only images for a certain pre-defined time period extending back from the current time when the current image was taken are considered in the search.

In step S250 for the search, the type of region of interest in the current image may be obtained by specific input provided by the user, or may be inferred from data such as stored in a medical database, or from the imaging protocol used. The type or region of interest for the prior imagery may likewise be inferred from data in the or other medial database. Specifically, the respective type of regions of interest are inferred through analysis of suitable contextual or metadata, such as clinical indication or imaging purpose information encoded into the historic imaging protocol used or as encoded in medical records. In addition or instead, the score localizers for localized IQ assessment scores of the prior images are used to determine the regions of interest in the priors $I_0$.

The components of the system SYS may be implemented as one or more software modules, run on one or more general-purpose processing units PU such as a workstation associated with the imager IA, or on a server computer associated with a group of imagers.

Alternatively, some or all components of the system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the system SYS may be implemented in both, partly in software and partly in hardware.

The different components of the system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. System (SYS) for imaging support, comprising:

an input interface (IN) for receiving an input image ($I_1$) of a patient acquired by an imaging apparatus (IA), the said input image previously assessed by an image quality assessment module (IQM) and awarded a first image quality, IQ, score (q); and

an imaging triaging logic (TL), configured to retrieve from an image database (DBI) of prior assessed images, a corresponding image ($I_0$) that corresponds to the said input image, the said corresponding image being a prior image ($I_0$) of said patient or of a similar patient, and the imaging triaging logic (TL) to provide a decision, based on the first IQ score and a second IQ score of the corresponding image ($I_0$), if any, whether or not to acquire a new image ($I_2$) of the patient by the imaging apparatus (IA).

2. System of claim 1, comprising a visualizer component (VC) to indicate i) whether or not such a corresponding image has been found, and/or ii) the IQ second score of said corresponding image, if found, and/or iii) an indication of the decision by the imaging triaging logic (TL).

3. System of claim 2, the visualizer (VC) to visualize, on a display device (DD), the said corresponding image.

4. System of any one of the previous claims, comprising a user interface (UI) configured to receive a user request for the imaging apparatus to acquire the said new image.

5. System of any one of previous claims, wherein, if no such corresponding image can be found, or if the second IQ score of the corresponding image does not exceed the first score, the triaging logic to decide that such a new image is to be acquired, and to i) issue a control signal to the imaging apparatus to effect a re-acquisition of the new image of the patient or ii) effect indicting to the user of the imaging apparatus a recommendation to acquire the new image.

6. System of any one of previous claims, wherein the imaging triaging logic (TL) is to retrieve the corresponding image from within a predefined prior time interval.

7. System of any one of the previous clams, wherein the time interval is dependent on any or more of: patient status, imaging protocol.

8. System of any one of the previous claims, wherein the retrieved corresponding image ($I_0$) is one having a region of interest t least overlapping with the region of interest in the current image.

9. System of any of the previous claims, wherein the decision by the imaging triaging logic (TL) is one of not to acquire the new image ($I_2$), if the second score is larger than the first score and/or wherein the second score is larger than a pre-defined minimal IQ score ($Q$).

10. System of any one of the previous clams, wherein the first and/or second IQ score is localizable in the respective image ($I_1$, $I_0$).

11. System of any one of the previous clams, wherein the decision by the logic (TL) is further based on a clinical indication in respect of the current image and/or of the prior image.

12. Computer-implemented method of imaging support, comprising:

receiving (220,210) an input image of a patient acquired by an imaging apparatus (IA), the said input image previously assessed by an image quality assessment module (IQM) and awarded a first image quality, IQ, score; retrieving (240) from an image database of prior assessed images, a corresponding image that corresponds to the said input image, the said corresponding image being a prior image of said patient or of a similar patient; and deciding (S270, S280), based on the IQ score and a second IQ score of the corresponding image, if any, whether or not to acquire a new image of the patient by the imaging apparatus (IA).

13. An imaging arrangement (AR), comprising the system (SYS) of any one of the previous claims, and the imaging apparatus (IA), the imaging apparatus being preferably of the medical X-ray type.

14. A computer program element, which, when being executed by at least one processing unit, is adapted to cause the processing unit (PU) to perform the method as per claim 12.

15. At least one computer readable medium having stored thereon the program element of claim 14.

**FIG. 1**

EP 4 134 972 A1

**FIG. 2**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 758 015 A1 (KONINKLIJKE PHILIPS NV [NL]) 30 December 2020 (2020-12-30) * paragraphs [0001], [0033] – [0036], [0045], [0053], [0066] * ----- | 1-15 | INV. G16H30/40 G16H40/63 G06N3/08 |
| X | US 2010/086189 A1 (WANG XIAOHUI [US] ET AL) 8 April 2010 (2010-04-08) * paragraphs [0012], [0038] * ----- | 1-15 | |
| X | US 2019/150857 A1 (NYE KATELYN ROSE [US] ET AL) 23 May 2019 (2019-05-23) * paragraph [0091] * ----- | 1-15 | |

TECHNICAL FIELDS
SEARCHED      (IPC)

G16H
G06N

The present search report has been drawn up for all claims

| Place of search | Date of completion of the search | Examiner |
|---|---|---|
| **Munich** | **27 January 2022** | **Reinbold, Bernhard** |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
..........................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 19 1175

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2022

| Patent document cited in search report | | Publication date | Patent family member(s) | | Publication date |
|---|---|---|---|---|---|
| EP 3758015 | A1 | 30-12-2020 | EP | 3758015 A1 | 30-12-2020 |
| | | | WO | 2020260540 A1 | 30-12-2020 |
| US 2010086189 | A1 | 08-04-2010 | US | 2010086182 A1 | 08-04-2010 |
| | | | US | 2010086189 A1 | 08-04-2010 |
| US 2019150857 | A1 | 23-05-2019 | CN | 109817304 A | 28-05-2019 |
| | | | EP | 3489964 A1 | 29-05-2019 |
| | | | JP | 2019093137 A | 20-06-2019 |
| | | | US | 2019150857 A1 | 23-05-2019 |
| | | | US | 2021015433 A1 | 21-01-2021 |

EPO FORM P0459

For more details about this annex : see Official Journal of the European Patent Office, No. 12/82